# EUROPEAN PATENT APPLICATION

(11) **EP 1 818 011 A2**
(43) Date of publication of application: **15.08.2007**
(21) Application number: 07102016.8
(22) Date of filing: 09.02.2007
(51) Int. Cl.: A61B 5/0245

(54) **System and method for monitoring a heartbeat**

(30) Priority: 09.02.2006 NL 1031117
(71) Applicant: HCS Cardio Systemen, 5673 MJ Nuenen (NL)
(72) Inventor: Hulsbergen, Stephan Arend, 5673 MJ, Nuenen (NL)
(74) Representative: Bakker, Hendrik

(57) **Abstract**

The invention relates to a system for monitoring a heartbeat, consisting of a sensor for generating an electrical signal representing a heartbeat (4), as well as a processing unit for deriving from this electrical signal and generating the numerical values representing the heartbeat. In addition, the processing unit comprises a processor provided with a program suitable for determining, on the basis of the electrical signal representing the heartbeat, whether a defibrillator must be set into operation.

## Description

The invention relates to a system for monitoring a heartbeat, comprising a sensor for generating an electrical signal representing a heartbeat, as well as a processing unit for deriving from this electrical signal and generating the numerical values representing the heartbeat.

Systems of this type are known. They are for instance used by sportspeople who wish to optimize and record their heartbeat frequency therewith. The sensor generally consists of a transmitting unit which transmits transmission signals in the rhythm of the heartbeat, provided with two electrodes which are placed on either side of the sternum and which are held in place using a chest strap in elastic form. The processing unit generally consists of a wristwatch in which a receiving unit is accommodated, in addition to a processor which determines the frequency of the transmission signals and shows them on a display.

The system according to the invention serves a completely different purpose. It serves specifically for use in combination with a known defibrillator. In the recent past defibrillators have been developed which can be set into operation by a layperson when someone has suspected cardiac arrest or ventricular fibrillation. The defibrillator is then arranged and independently determines whether it is worthwhile administering electrical shocks. If this is the case, it then delivers a number of electrical shocks, wherein it monitors repeatedly whether these electrical shocks are having the intended effect.

The problem is that the layperson does not always know or realise that someone is suffering from cardiac arrest or ventricular fibrillation. It is thus possible to envisage, for instance in the case of a couple, a heart patient dying in his/her sleep while the partner is unaware of this and will therefore not apply a possibly available defibrillator. The present invention obviates this drawback and has the feature that the processing unit also comprises a processor provided with a program suitable for determining, on the basis of the electrical signal representing the heartbeat, whether a defibrillator must be set into operation. The sensor can for instance again consist of a transmitting unit which transmits transmission signals in the rhythm of the heartbeat, provided with two electrodes which are placed on either side of the sternum and which are held in place with a chest strap in elastic form, but it can also take other, less cumbersome forms, such as for instance an optical connection which is arranged on a finger and which detects the pulsating blood flow. The processing unit can again then consist of for instance a wristwatch, whereby a wireless connection is unnecessary, although it can also be formed by for instance an alarm or telephone accommodating a receiver for transmission signals.

A defibrillator is known from WO 2004/105869 which can be worn permanently and which can independently determine whether defibrillation has to be carried out. If this is the case, it can also independently deliver electrical shocks. If a defibrillator is already available, the system according to the invention is then a more advantageous solution.

A favourable embodiment of the inventive system has the feature that the processing unit is adapted to generate an auditory and/or visual warning signal when a defibrillator must be set into operation. This embodiment can be combined in excellent manner with a processing unit embodied as alarm or telephone, in which an auditory warning signal is after all already available.

A practical embodiment has the feature that the sensor and/or processing unit are provided with means for portable fastening to the body.

In a further practical embodiment the sensor and/or processing unit are provided with their own energy source, such as a battery or a photoelectric cell. In this embodiment the user has a higher measure of freedom.

In a further alternative practical embodiment, sensor and processing unit are wirelessly connected to each other.

A further advantageous embodiment has the feature that the processing unit is provided with connecting means with which a connection to for instance a central reporting station can be realized. A warning signal can likewise then be generated there. At the central reporting station the signals representing the heartbeat are preferably also evaluated, whereafter instructions can be issued and for instance a properly equipped ambulance can be utilized.

A further favourable embodiment has the feature that the connecting means comprise a connection for a computer, for instance a USB connection. The computer can then also evaluate the signals representing the heartbeat, whereafter instructions can be issued and a properly equipped ambulance can for instance be requested via an internet connection.

A favourable alternative embodiment has the feature that the connecting means comprise a telephone with which an emergency number can be called, whereafter the processing unit leaves a message.

The invention also relates to a sensor or processing unit as part of a system as specified in the foregoing paragraphs.

The invention also relates to a method for processing electrical signals representing a heartbeat, wherein the numerical values representing the heartbeat are derived from the electrical signals representing the heartbeat. Such a method is applied in modern defibrillators which can be employed by a layperson, so effectively preventing unnecessary electrical shocks being administered to a patient. According to the present invention, the method is applied in order to monitor a heart patient before a defibrillator is placed. The inventive method therefore has the feature that an auditory and/or visual warning signal is generated when a numerical value representing the heartbeat lies outside a predetermined interval for a predetermined period of time.

A favourable realization of the inventive method has the feature that an auditory and/or visual warning signal is generated when a heartbeat frequency lies outside a predetermined frequency interval for a predetermined period of time or when a variance in the heartbeat frequency is greater than a predetermined value for a predetermined period of time.

A further favourable realization of the inventive method has the feature that a warning signal is also generated to a computer together with the electrical signals representing the heartbeat. Using a program stored therein, the computer can then once again evaluate the signals representing the heartbeat and give instructions. It can also alert a central reporting station, for instance via an internet connection.

A further favourable realization of the inventive method has the feature that a signal is also generated to a telephone, which is programmed such that it selects a predetermined telephone number and passes on a message. The telephone is preferably adapted such that it also transmits the electrical signals representing the heartbeat.

The invention will now be further elucidated with reference to the following figures, wherein:
- Fig. 1: shows a possible embodiment of a system according to the invention;
- Fig. 2: shows an alternative embodiment of a system according to the invention;
- Fig. 3: shows the operation of the device of Fig. 2 in the form of a flow diagram.

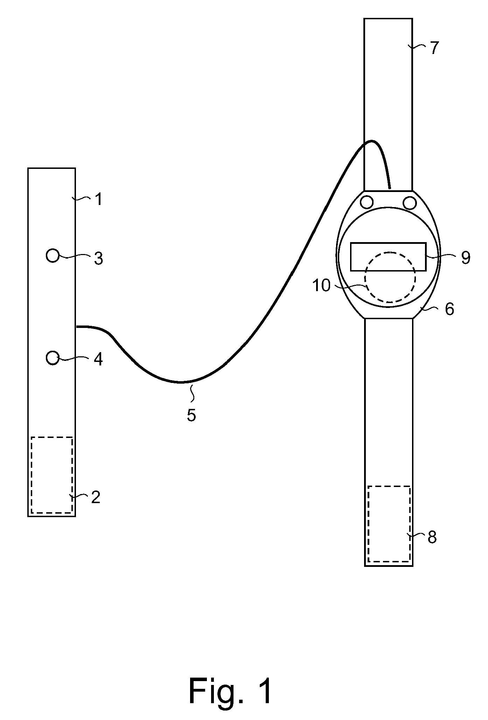

Fig. 1 shows a possible embodiment of a system according to the invention. The system comprises a flexible strap 1 which can be fastened around a finger using a velcro tape closure 2 and which is provided with a light-emitting diode 3 and a light-sensitive detector 4, this such that in the position of use the light from light-emitting diode 3 can reach light-sensitive detector 4, wherein the amount of light depends on the momentary blood circulation in the finger, so that the heartbeat can be derived from the electrical signal generated by light-sensitive detector 4. Using a releasable electrical connection 5 the strap 1 is connected to a watch 6 which can be fastened around the wrist using a strap 7 and using a velcro fastening 8. Watch 6 comprises a battery (not shown) and a processor (not shown) which processes the electrical signal from light-sensitive detector 4 in per se known manner such that a heartbeat frequency can for instance be shown on a display 9, but also such that a situation in which defibrillation is necessary can be detected. In this case a piezoelectric beeper 10 is for instance switched on, which makes a member of the household aware that defibrillation must take place.

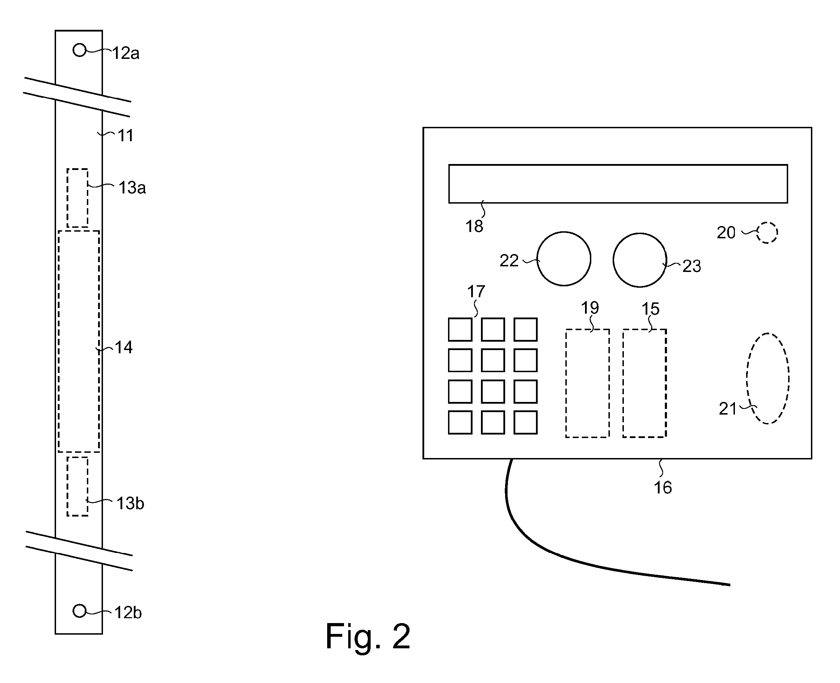

Fig. 2 shows an alternative embodiment of a system according to the invention. The system comprises a flexible chest strap 11 which can be fastened around the chest using a closure 12a,12b and which is provided with two electrodes 13a,13b, a per se known transmitter 14 and a battery (not shown), this such that in the position of use an electrical signal coming from the heart is received, amplified and transmitted in per se known manner by electrodes 13a,13b. The transmitted signal is received by a receiver 15 in a telephone 16 embodied as a hand-free set. Telephone 16 further comprises a keyboard 17, a display 18 and a processor 19, as well as a microphone 20 and a loudspeaker 21. Processor 19 receives the electrical signal received by receiver 15 and analyses it in a per se known manner such that a situation wherein defibrillation is necessary can be detected. If this is the case, spoken instructions are given to a member of the household via loudspeaker 21. If the household member concludes that there is indeed an alarming situation, he/she then presses a button 22, whereafter he/she receives further instructions via loudspeaker 21. An emergency number is moreover called via telephone 16, wherein the household member can inform someone of the situation and can receive instructions in hand-free manner. If desired, processor 19 can also pass on the electrical signals from the heart via the telephone. If the household member concludes that there has been a false alarm, he/she then presses a button 23, whereafter processor 19 once again begins to monitor the heartbeat.

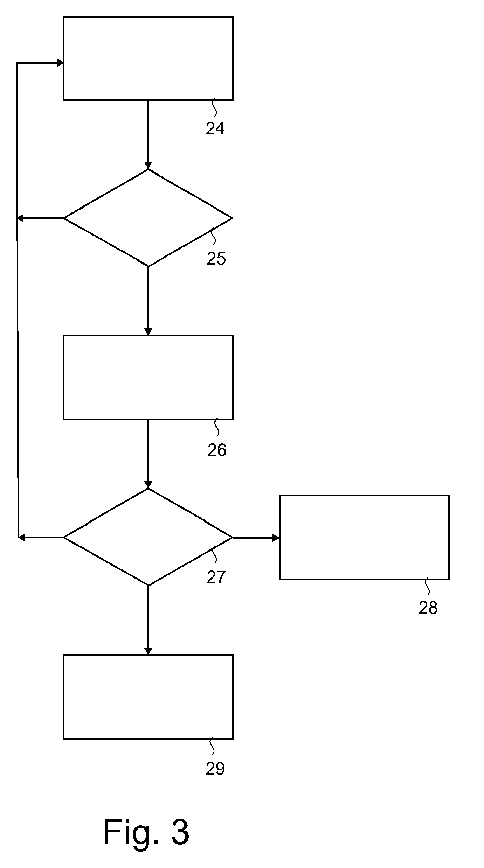

Fig. 3 shows the operation of the device of Fig. 2 in the form of a flow diagram. Block 24 herein shows the heartbeat measurement function, the outcome of which is continuously evaluated in block 25. If the heartbeat is for instance lower than a predetermined minimum value or higher than a predetermined maximum value, block 26 is then activated, which entails a warning being generated and for instance spoken instructions being given, such as "check breathing", "check circulation", "press an alarm button if either of the two is not correct", "press a reset button if both are correct". Block 27 waits for the alarm button or the reset button to be pressed. If the reset button is pressed, block 24 is then reactivated. If the alarm button is pressed, block 28 is then activated and spoken instructions are given, such as "take the defibrillator and place it". A hand-free line is moreover opened to the emergency number, usually 112. If no connection to the emergency number is obtained, there then for instance follows the announcement "connection to the emergency number cannot be made". It is then for instance possible to attempt to reach the emergency number or an alternative number in another way. Finally, if no button is pressed within a predetermined time, a connection is then made in block 29 to for instance an emergency centre or a family member or to neighbours, wherein a spoken message can be issued.

The invention has for its object to provide a consumer product for the purpose of detecting heart failure of the user and for then generating a warning signal using the technical measures as stated in claim 1 and/or 2.

The invention is further based on the inventive concept that the system can be fastened to the body of the user in portable manner, wherein the inconvenience experienced by the user is limited to a minimum. The system can also be provided for this purpose with other fastening means than said wristband or chest strap, such as for instance an ankle band or ring. The dimensions and the weight of the portable part of the system are comparable to those of a Walkman. The wearer comfort can be further enhanced by giving the system a wireless form and providing it with an internal energy source.

The system is intended particularly for use at night.

The system is of course not limited to the described and shown preferred embodiments, but extends to any embodiment which falls within the scope of protection as defined in the claims, as seen in light of the foregoing description and associated drawings.

## Claims

1. System for monitoring a heartbeat, comprising a sensor for generating an electrical signal representing a heartbeat, as well as a processing unit for deriving from this electrical signal and generating the numerical values representing the heartbeat, **characterized in that** the processing unit also comprises a processor provided with a program suitable for determining, on the basis of the electrical signal representing the heartbeat, whether a defibrillator must be set into operation.

2. System as claimed in claim 1, **characterized in that** the processing unit is adapted to generate an auditory and/or visual warning signal when a defibrillator must be set into operation.

3. System as claimed in claim 1 or 2, **characterized in that** the sensor and/or the processing unit are provided with means for portable fastening to the body.

4. System as claimed in claims 1-3, wherein the sensor and/or the processing unit are provided with an internal energy source, such as a battery or a photoelectric cell.

5. System as claimed in claims 1-4, wherein the sensor the sensor and the processing unit are connected wirelessly.

6. System as claimed in claims 1-5, **characterized in that** the processing unit is provided with connecting means with which a connection to for instance a central reporting station can be realized.

7. System as claimed in claim 6, **characterized in that** the connecting means are wireless.

8. System as claimed in claim 6 or 7, **characterized in that** the connecting means comprise a connection for a computer.

9. System as claimed in claim 6 or 7, **characterized in that** the connecting means comprise a telephone.

10. Sensor or processing unit as part of a system as claimed in any of the claims 1-9.

11. Method for processing electrical signals representing a heartbeat, wherein the numerical values representing the heartbeat are derived from the electrical signals representing the heartbeat, **characterized in that** an auditory and/or visual warning signal is generated when a numerical value representing the heartbeat lies outside a predetermined interval for a predetermined period of time.

12. Method as claimed in claim 11, **characterized in that** an auditory and/or visual warning signal is generated when a heartbeat frequency lies outside a predetermined frequency interval for a predetermined period of time or when a variance in the heartbeat frequency is greater than a predetermined value for a predetermined period of time.

13. Method as claimed in claim 11 or 12, **characterized in that** a warning signal is also generated to a computer together with the electrical signals representing the heartbeat.

14. Method as claimed in claim 11, **characterized in that** a signal is also generated to a telephone, which is programmed such that it selects a predetermined telephone number and passes on a message.
